# EUROPEAN PATENT APPLICATION

(11) **EP 1 854 611 A1**
(43) Date of publication of application: **14.11.2007**
(21) Application number: 06009559.3
(22) Date of filing: 09.05.2006
(51) Int. Cl.: B29C 45/14, A61B 17/17, B29C 65/42

(54) **Connection between two component parts by means of injection molding, auxiliary attachment for medical purposes**

(71) Applicant: Stryker Trauma GmbH, 24232 Schönkirchen/Kiel (DE)
(72) Inventor: Cremer, Axel, 23795 Fahrenkrog (DE)
(74) Representative: Kopf, Korbinian Paul

(57) **Abstract**

It is described a joining assembly (260) of two component parts (230, 240). The joining assembly (260) includes a first component part (230) comprising a contour (236) and a second component part (240) comprising a cavity (245). The first component part (230) is arranged relative to the second component part (240) in such a manner, that in between the contour (236) and the cavity (245) there is formed a hollow space. The joining assembly (260) further includes a connecting material, which is filled into the hollow space. After the connecting material has been filled into the hollow space, the connecting material is hardened. Preferably, undercuts (237, 238) are provided in order to ensure a tight and permanent connection between the two component parts (230, 240). The joining assembly (260) is in particular suitable for connecting two different component parts (230, 240), which are made from different materials such as metal and an X-ray transparent plastic, respectively. The joining assembly (260) may be used for medical purposes for connecting a CFK aiming device (140) with an implant (110) for treating bone fractures, whereby a precise spatial relative position of the aiming device (140) with respect to the implant (110) is established.

## Description

### Field of invention

The present invention relates to the field of mechanically connecting two different component parts with each other. In particular the present inventions relates to a joining method for permanently connecting two different component parts with each.

The present invention further relates to a joining assembly of two different component parts.

Further, the present invention relates to an auxiliary attachment for medical purposes, in particular for osteosynthesis auxiliary for the treatment of bone fractures, whereby the auxiliary attachment comprises a joining assembly as described above.

### Art Background

In order to allow for a reliable stabilization of a broken bone in its normal position, it is known to use special bone stabilizing implants. Such implants are for instance metal plates or intramedullary locking nails, which are made in particular from surgery steel. Plates and locking nails used for such purposes are usually fixed to the bone parts by means of threaded screws, which are driven into the bone tissue after so-called pre-drilled or pilot-drilled holes have been generated within the bone tissue. These pre-drilled holes allow for a reliable screwing procedure whereby the risk of further destroying the bone with the screw is significantly reduced.

In order to facilitate the drilling of these holes there are known so-called aiming devices, which work like a drilling jig. Thereby, an aiming device is detachably fixed to the implant in a spatial precise position.

Aiming devices are in particular used in connection with locking nails, which are driven into intra medullary tissue. Thereby, the position of a cross bore within the interlocking nail can be determined precisely. The cross bore is adapted to accommodate a fixing screw, which is driven crosswise through the corresponding bone section.

US 6,224,601 B1 discloses the use of an aiming device in an osteosynthesis auxiliary for the treatment of subtrochanteric, peritochanteric and femoral-neck fractures.

In order to provide for a precise pre drilling of holes the mechanical interface between locking nail and aiming device must comprise a high material strength in order to provide for a reliable and defined load transmission from the aiming device to the intramedullary locking nail. Thereby, both strengths and moments of force have to be transmitted.

In order to facilitate many surgeries an aiming device is made from a material being more or less transparent for X-rays within the diagnostically relevant spectral range between approximately 10 keV and 150 keV. Typically, the X-ray transparent material is a synthetic material.

When building up an aiming device such an X-ray transparent material has to be mechanically connected with a positioning arm of the aiming device, which positioning arm is typically made from a metallic material. Usually, for connecting these different materials with each other a special glue or adhesive is employed. However, this has the disadvantage that the stability of the glue decreases when the whole aiming device is repeatedly subjected to sterilization procedures like e.g. steam sterilization.

There may be a need for both a stable and long-lasting mechanically connection between two different component parts with each other.

### Summary of the Invention

This need may be met by the subject matter according to the independent claims. Advantageous embodiments of the present invention are described by the dependent claims.

According to a first aspect of the invention there is provided a joining method for mechanically connecting two component parts with each other, a first component part comprising a contour and a second component part comprising a cavity. The described method comprises (a) positioning the first component part and the second component part relative to each other in a predetermined position, whereby in between the contour and the cavity there is formed a hollow space, (b) injecting a connecting material into the hollow space and (c) hardening the connecting material.

This aspect of the invention is based on the idea that both a permanent and stable connection may be realized by inserting a connecting material into a hollow space, which is voluminously formed in between the contour and the cavity.

The contour may be any profile, which is adapted to spatially interact with the cavity. Preferably, the contour is a protrusion, which can be inserted into the cavity. Thereby, the shape of cavity is preferably adapted to the shape of the contour in such a manner that the hollow space is on the one hand as small as possible and on the other hand enough to accommodate a proper amount of connecting material. A proper amount of connecting material may provide for a stable and a mechanically very reliable connection.

However, the contour may also be an opening, which in connection with the cavity forms a common hollow space. Thereby, when connecting the two component parts, the connecting material penetrates into both the contour and the cavity. In other words, the connecting material, when hardened, represents a connecting member, which engages into both the contour and the cavity.

The connecting material is at the beginning fluid or clammy and becomes hard later on. The hardening procedure may be carried out e.g. by temporally heating up the connecting material. However, the hardening procedure might also proceed automatically e.g. by appropriate chemical reactions.

By contrast to known joining techniques, which are based on bonding or adhesion forces, the described joining method provides the advantage that a very durable, stable and reliable connection between the two parts may be formed. In particular, the connecting material may have a thermal expansion coefficient being similar to the thermal coefficients of the first and/or the second component part. Thereby, thermal stress may be reduced significantly.

Further, by contrast to known adhesives the connecting material may exhibit improved hygroscopic properties, i.e. the connecting material may be less hygroscopic such that an optimal disinfection procedure may be used for an aiming device without accounting for the stability of a adhesive.

Preferably, the connecting material is filled into the hollow space with high pressure. This may provide the advantage that no or only very little residual cavities will remain within the hollow space. Therefore, the mechanical connection is almost free of voids. In particular, when such a joined assembly of the two component parts is used for medical purposes or more specifically for surgeries, such voids would be very difficult to sterilize. Therefore, a corresponding joined assembly having no or only very few residual voids reduces the risk for infections, which risk is always existent in any type of surgery.

According to an embodiment of the invention, the step of injecting a connecting material into the hollow space is carried out by means of an injection molding procedure. This has the advantage that a well-known procedure can be used in order to establish a close and tight connection between the two component parts. Therefore, in order to find appropriate process parameters one can use the know how of the whole technical field of injection molding.

According to a further embodiment of the invention the contour and/or the cavity comprises an undercut. Providing an undercut has the advantage that a hardened filling material may engage respectively interlock with the undercuts such that a stable and permanent connection between the two component parts may be achieved.

According to a further aspect of the invention there is provided a joining assembly of two component parts. The joining assembly comprises (a) a first component part comprising a contour, (b) a second component part comprising a cavity, wherein the first component part is arranged relative to the second component part in such a manner that in between the contour and the cavity there is formed a hollow space, and (c) a connecting material, which is filled into the hollow space.

This aspect of the invention is based on the idea that both a force-fit (non-positive fit) and a form-fit (positive fit) connection between the two different component parts may be realized by inserting a connecting material into a hollow space, which is formed in between the contour and the cavity.

The hollow space may be any geometrical structure, which is adapted to receive the connecting material. The connecting material may be filled into the hollow space with high pressure. This may provide the advantage that no or only very little residual cavities will remain within the hollow space such that the mechanical connection is almost free of voids.

Preferably, the connecting material is a viscous fluid, which may be filled into the hollow space e.g. by means of an injection molding procedure. After the connecting material has been brought into the hollow space the material has to become solid. This can be achieved e.g. by temporally heating up the connecting material or by just waiting for a specified duration until the connecting material is automatically hardened e.g. by appropriate chemical reactions.

In order to provide for an effective injection process the connecting material may be injected through an injection channel. The injection channel may be provided within the first and/or the second component part.

According to an embodiment of the invention the contour is a flange. This has the advantage that the contour may be manufactured easily by means of standard milling techniques.

Preferably, the flange comprises a rotational symmetric shape. In this case the flange may be manufactured in particular easily by means of standard turning techniques on a lathe.

Preferably, the contour is formed and/or surface treated by means of known erosion techniques such as chemical erosion or spark erosion. Thereby, a geometrically precise body having smooth surfaces may be provided.

According to a further embodiment of the invention the flange is formed integrally with the first component part. This may provide the advantage that no extra connection between the first component part and the contour protruding therefrom is necessary. Further, the integral formation of the contour and the first component part has the advantage that the whole contour is mechanically very stable and reliable. Preferably, the flange is welded on the first component such that both the flange and the first component may be manufactured in a manner being appropriate for the respective component part.

According to a further embodiment of the invention the contour and/or the cavity comprises an undercut. As has already been mentioned above, providing an undercut has the advantage that a hardened filling material may engage respectively interlock with the undercuts such that a stable and permanent connection between the two component parts may be achieved.

According to a further embodiment of the invention the undercut comprises a circumferential groove. Preferably, the circumferential groove is formed within the outer surface of a flange having a cylindrical shape. This has the advantage that also the circumferential groove may be formed easily by means of standard turning techniques. However, alternatively the flange may also have any other shapes such as the shape of a cuboid.

Furthermore, the circumferential groove may be used as a raceway for the connecting material when the two component parts are connected by means of an injection molding procedure whereby a fluid connecting material is inserted into the hollow space.

According to a further embodiment of the invention the undercut comprises a longitudinal groove. This may provide the advantage that an unwanted torsion or twist between the two component parts may be prevented even if the contour and/or the cavity comprise a cylindrical shape.

It has to be mentioned that also the longitudinal groove may be used as a raceway for the liquid connecting material.

According to a further embodiment of the invention the first component part is made at least partially from metal. In particular when the joining assembly is used for medical purposes the first component part preferably is made from titan and/or steel, whereby the steel is preferably a high-alloy material.

According to a further embodiment of the invention the second component part is made at least partially from a material being transparent for X-rays. In this respect X-ray transparent means that the X-ray attenuation of the material is so small that X-ray imaging is still possible with X-rays traversing the second component.

The material from which the second component part is made is in particular transparent for X-rays within the diagnostically relevant spectral energy range. Typically, this range is defined by a lower X-ray energy of approximately 60 keV and by a higher X-ray energy of approximately 140 keV.

According to a further embodiment of the invention the second component part is made at least partially from a synthetic material. Due to the improvement of synthetic materials and the corresponding manufacturing processes for synthetic materials both light and precise second component parts may be manufactured, which are joinable with a first component part made from a different material. Anyway, a mechanically stable and durable connection may be formed with these materials.

According to a further embodiment of the invention the second component part is made at least partially from a laminated material. This has the advantage that the second component part may be made from a synthetic material having a high mechanical stability.

According to a further embodiment of the invention the second component part is made at least partially from a material being reinforced with carbon fibers. In particular, a CFK material may be used. CFK provides the advantage that it represents a comparatively light material, which nonetheless is mechanically very stable, very durable and insensitive to physical and/or chemical treatment used for disinfection procedures.

According to a further aspect of the invention there is provided an auxiliary attachment for medical purposes, in particular for osteosynthesis auxiliary for the treatment of bone fractures. The described auxiliary attachment comprises (a) a positioning arm, which is adapted to be mechanically coupled to a bone stabilizing implant, and (b) an aiming device, which is adapted to be mounted to the positioning arm in a spatially defined position with respect to the bone stabilizing implant. Thereby the positioning arm and/or the aiming device comprises a joining assembly as set forth in any one of the above described embodiments of the joining assembly.

This aspect of the invention is based on the idea that the described both force-fit (non-positive fit) and form-fit (positive fit) connection between the two different component parts may be used beneficially for surgery aiming devices, where a precise and durable connection between a metal component part and a synthetic X-ray transparent component part is necessary.

The employed joining assembly provides the advantage that an auxiliary attachment may be provided, which is not only mechanically very stable and durable but which is also insensitive to chemical and physical treatments used for disinfection purposes. Therefore, common known disinfection procedures may be carried out with the auxiliary attachment without taking care of the thermal and chemical stability of glues, adhesives or other materials. By contrast to a glue, the injected connecting material has the advantage that all known disinfection procedures do not affect the stability of the mechanical connection and may be used in order to provide for a reliably disinfection. Therefore, the described joining assembly may decrease the risk for patients of getting infected because of a non-perfect disinfection of the auxiliary attachment.

With the help of the described auxiliary attachment a bone stabilizing implant may be fixed very precisely to a broken bone or to fragments of a broken bone. Thereby, the aiming device may be used in order to precisely drill holes into the bone, whereby each hole is formed exactly at the proper position with respect to a corresponding through hole in the bone stabilizing implant. Therefore, the aiming device may be seen as a drilling jig assisting a surgeon for precisely drilling holes into the bone of a patient at the appropriate locations.

Typically, the bone stabilizing implant is a plate or an intramedullary interlocking nail, which may be inserted into the bone marrow.

It has to be noted that embodiments of the invention have been described with reference to different subject matters. In particular, some embodiments have been described with reference to method type claims whereas other embodiments have been described with reference to apparatus type claims. However, a person skilled in the art will gather from the above and the following description that, unless other notified, in addition to any combination of features belonging to one type of subject matter also any combination between features relating to different subject matters, in particular between features of the method type claims and features of the apparatus type claims is considered to be disclosed with this application.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

### Brief Description of the Drawings

Figure 1 shows an aiming device being attached to an interlocking nail according to a preferred embodiment of the invention.
Figure 2a shows an exploded front view of a join connection between a metallic guide block and a CFK aiming device.
Figure 2b shows an exploded top view of the join connection between a metallic guide block and a CFK aiming device shown in Figure 2a.

### Detailed Description

The illustration in the drawing is schematically. It is noted that in different figures, similar or identical elements are provided with the same reference signs or with reference signs, which are different from the corresponding reference signs only within the first digit.

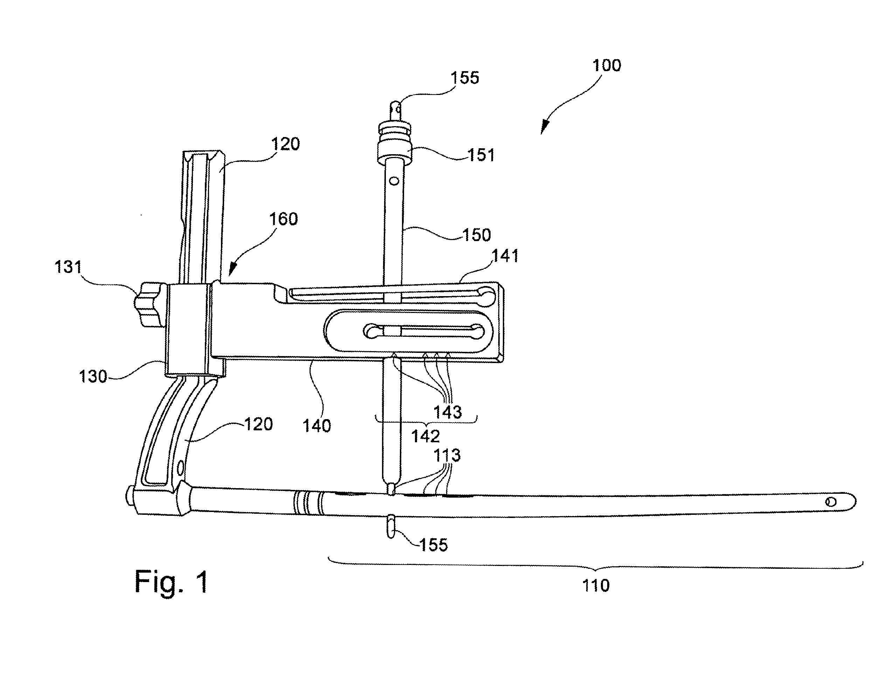

Figure 1 shows an auxiliary attachment 100 preferably used for surgery purposes. The auxiliary attachment 100 comprises an intramedullary interlocking nail 110, which is used in order to fix a broken bone in its anatomical correct position. Thereby, the nail 110 is longitudinally inserted into medullar tissue of the broken bone. In order to prevent from a disarrangement of the nail 110 with respect to the broken bone the nail 110 is provided with transverse holes 113, which are adapted to accommodate fixing screws.

The auxiliary attachment 100 further comprises a positioning arm 120 and a metallic guide block 130. Attached to the guide block 130 is an aiming device 140, which is made from a synthetic material being transparent for X-rays in the diagnostically relevant spectral range. According to the embodiment described herewith the aiming device 140 is made from CFK. When the whole auxiliary attachment 100 is assembled as depicted in Figure 1, the aiming device 140 is positioned with respect to the interlocking nail 110 in a spatially precisely defined location.

The aiming device 140 comprises several through holes 143 each representing a drilling jig 142. The through holes 143 are arranged in such a manner that they correspond with the transverse holes 113.

The aiming device 140 is very helpful for a surgery wherein the nail 110 is inserted into a broken bone. Thereby, by using the drilling jig 142 an operator is capable of precisely drilling holes into the bone, whereby each hole is formed exactly at the proper position with respect to a corresponding transverse hole 113.

The distance between the aiming device 140 and the nail 110 may be adjusted by shifting the guide block 130 along the positioning arm 130. When the proper distance is established, a locking screw comprising a handle 131 can be tightened. Thereby, an adjusted distance between the aiming device 140 and the nail 110 can be maintained.

The through holes 143 have an inner diameter, which corresponds to the outer diameter of a guide shaft 1 S0. The guide shaft 150 can be inserted into one of the through holes 143 with an appropriate lateral tolerance. In order to ensure a self-locking of the guide shaft 150 within the aiming device 140 there is provided a clamping element 141at the aiming device 140. According to the embodiment described here, the clamping element 141 represents a resilient tongue. When the free end of the clamping element 141 is pressed towards the main body of the aiming device 140, the guide shaft 150 may be easily shifted. When the clamping element 141 is released, the longitudinal position of the guide shaft 150 with respect to the aiming device is fixed.

In order to allow for an easy handling the guide shaft 150 is provided with knurled nut 151.

The guide shaft 150 is a hollow shaft comprising a cylindrical passage (not depicted). The cylindrical passage is adapted to accommodate a drill bit 155. The drill bit 155 typically is used to pre-drill or pilot-drill holes within the bone tissue. These pre-drilled holes allow for a reliable screwing procedure whereby the risk of further destroying the bone with the screw is significantly reduced.

The guide block 130 and the aiming device 140 are mechanically connected with each other by means of a join connection 160, which is formed at the intersection between the metallic guide block 130 and the CFK aiming device 140.

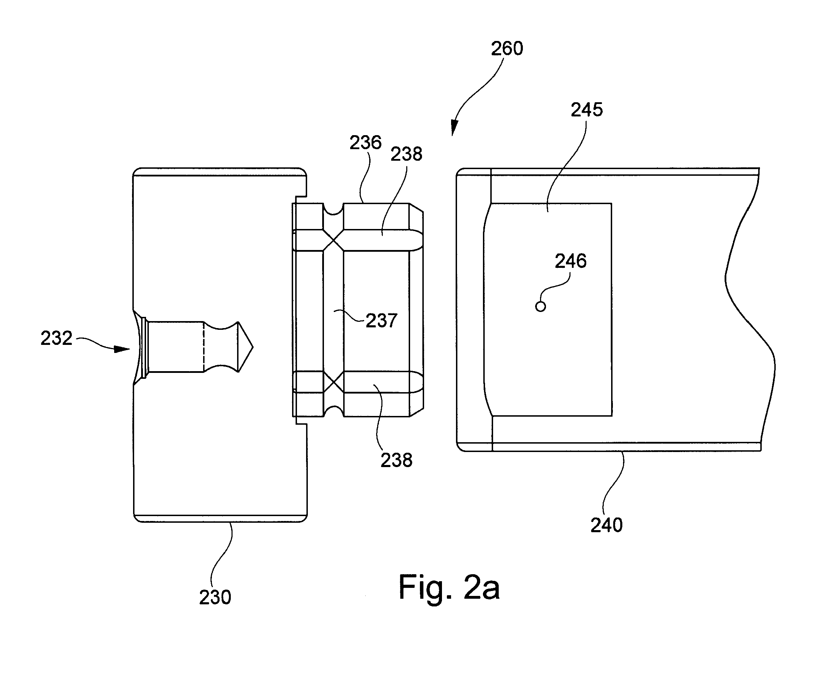

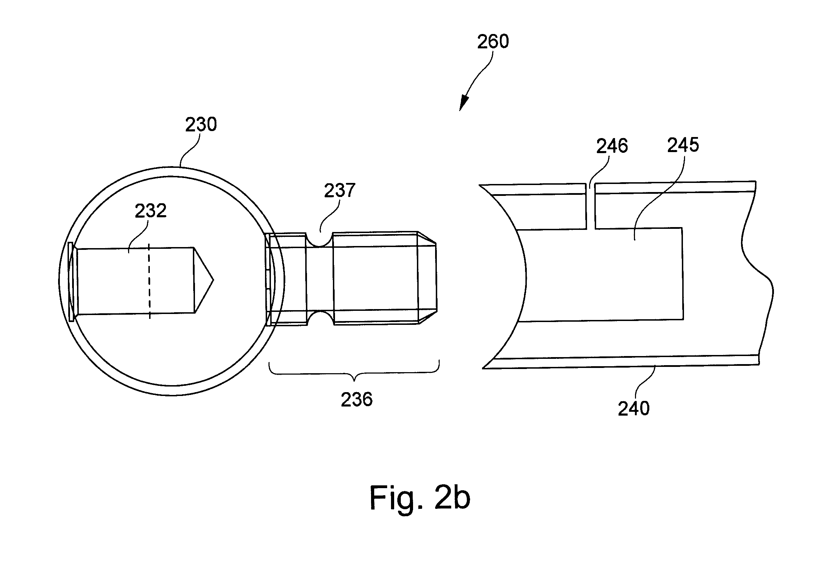

Figures 2a and 2b show the join connection 160 in more detail. Thereby, figure 2a shows an exploded front view of the join connection 160, which is now denominated with reference numeral 260. Figure 2b shows an exploded top view of the join connection 260 between the metallic guide block 230 and the aiming device 240 made from CFK.

The join connection 260 between the metallic guide block 230 representing a first component part and the CFK aiming device 240 representing a second component part is realized by means of a injection molding procedure. Thereby, the first component part 230 and the second component part 240 are positioned relative to each other in a predetermined position, whereby a contour 236 of the first component part 230 is inserted into a cavity 245 of the second component part 240. Thereby, a hollow space is formed in between the contour 236 and the cavity 245.

According to the embodiment described here the contour is a flange 236. The flange 236, which is also a metallic member having a cylindrical or preferably a cuboid shape, is build up on the first component part 230 by means of a welded connection. Therefore, the first component part 230 and the flange 236 are formed integrally.

For inserting a fluid representing a connecting material into the hollow space formed in between the contour 236 and the cavity 245 an insertion channel 246 is provided. The material transport of the fluid connecting material is supported by a circumferential grove 237 and two longitudinal grooves 238, which are formed within the outer surface of the contour 236. These grooves represent a raceway for the connecting material when it is inserted into the hollow space being formed in between the contour 236 and the inner walls of the cavity 245.

However, the grooves 237 and 238 have a further important function because they represent undercuts. After the injection molding procedure has been completed and the inserted connecting material has been hardened, the undercuts 237 and 238 ensure that the first component part 230 and the second component part 240 maintain in the correct relative position with respect to each other. Thereby, the undercut 237 primarily ensures that the two component parts 230 and 240 are not disconnected. The undercuts 238 primarily ensure that the relative angular position between the two component parts 230 and 240 with respect to the longitudinal of the flange 236 is maintained.

It has to be mentioned that of course also undercuts may be provided within the cavity 245. However, according to the embodiment described here, such undercuts are not necessary because the synthetic connecting material and the CFK material, which forms the second component part, merge with each other such that a tight connection between the connecting material and the CFK material is automatically established at least after the connecting material has been hardened.

As has already been mentioned above, the guide block respectively the first component part may be fixed to the positioning arm be means of a locking screw. Therefore, the guide block 230 comprises a threaded bore 232, which is adapted to accommodate the locking screw.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

In order to recapitulate the above described embodiments of the present invention one can state:
It is described a joining assembly 260 of two component parts 230, 240. The joining assembly 260 includes a first component part 230 comprising a contour 236 and a second component part 240 comprising a cavity 245. The first component part 230 is arranged relative to the second component part 240 in such a manner, that in between the contour 236 and the cavity 245 there is formed a hollow space. The joining assembly 260 further includes a connecting material, which is filled into the hollow space. After the connecting material has been filled into the hollow space, the connecting material is hardened. Preferably, undercuts 237, 238 are provided in order to ensure a tight and permanent connection between the first component part 230 and the second component part 240. The joining assembly 260 is in particular suitable for connecting two different component parts 230, 240, which are made from different materials such as metal and an X-ray transparent plastic, respectively. The joining assembly 260 may be used for medical purposes for connecting a CFK aiming device 140 with an implant 110 for treating bone fractures, whereby a precise spatial relative position of the aiming device 140 with respect to the implant 110 is established.

### List of reference signs:

- 100: auxiliary attachment
- 110: intramedullary nail
- 113: transverse holes
- 120: positioning arm
- 130: first component part / guide block
- 131: handle of a locking screw
- 140: second component part / aiming device
- 141: clamping element / resilient tongue
- 142: drilling jig
- 143: through hole
- 150: guide shaft
- 151: knurled nut
- 155: drill bit
- 160: join connection

- 230: first component part / guide block
- 232: bore for locking screw
- 236: contour / flange
- 237: circumferential groove / undercut
- 238: longitudinal groove / undercut
- 240: second component part / aiming device
- 245: cavity
- 246: injection channel
- 260: join connection

## Claims

1. A joining method for mechanically connecting two component parts (230, 240) with each other, a first component part (230) comprising a contour (236) and a second component (240) part comprising a cavity (245), the method comprising
positioning the first component part (230) and the second component part (240) relative to each other in a predetermined position, whereby in between the contour (236) and the cavity (245) there is formed a hollow space,
injecting a connecting material into the hollow space and
hardening the connecting material.

2. The joining method as set forth in claim 1, wherein
injecting a connecting material into the hollow space is carried out by means of an injection molding procedure.

3. The joining method as set forth in any one of the claims 1 to 2, wherein
the contour (236) and/or the cavity (245) comprises an undercut (237, 238).

4. A joining assembly of two component parts (230, 240), the joining assembly comprising
a first component part (230) comprising a contour (236),
a second component part (240) comprising a cavity (245), wherein the first component part (230) is arranged relative to the second component part (240) in such a manner, that in between the contour (236) and the cavity (245) there is formed a hollow space, and
a connecting material, which is filled into the hollow space.

5. The joining assembly as set forth in claim 5, wherein
the contour is a flange (236).

6. The joining assembly as set forth in claim 6, wherein
the flange (236) is formed integrally with the first component part (230).

7. The joining assembly as set forth in any one of the claims 4 to 6, wherein
the contour (236) and/or the cavity (245) comprises an undercut (237, 238).

8. The joining assembly as set forth in claim 7, wherein
the undercut comprises an circumferential groove (237).

9. The joining assembly as set forth in any one of the claims 7 to 8, wherein
the undercut comprises a longitudinal groove (238).

10. The joining assembly as set forth in any one of the claims 4 to 9, wherein
the first component part (230) is made at least partially from metal.

11. The joining assembly as set forth in any one of the claims 4 to 10, wherein
the second component part (240) is made at least partially from a material being transparent for X-rays.

12. The joining assembly as set forth in any one of the claims 4 to 11, wherein
the second component part (240) is made at least partially from a synthetic material.

13. The joining assembly as set forth in any one of the claims 4 to 12, wherein
the second component part (240) is made at least partially from a laminated material.

14. The joining assembly as set forth in any one of the claims 4 to 13, wherein
the second component part (240) is made at least partially from a material being reinforced with carbon fibers.

15. An auxiliary attachment (100) for medical purposes, in particular for osteosynthesis auxiliary for the treatment of bone fractures, the auxiliary attachment comprising
a positioning arm (120), adapted to be mechanically coupled to a bone stabilizing implant (110), and
an aiming device (140), adapted to be mounted to the positioning arm (120) in a spatially defined position with respect to the bone stabilizing implant (110), wherein
the positioning arm (120) and/or the aiming device (140) comprises a joining assembly (160) as set forth in any one of the claims 4 to 15.
